(19) Europäisches Patentamt
European Patent Office
Office européen des brevets

(11) EP 1 404 373 B1

(12) EUROPÄISCHE PATENTSCHRIFT

(45) Veröffentlichungstag und Bekanntmachung des Hinweises auf die Patenterteilung:
**17.09.2008 Patentblatt 2008/38**

(21) Anmeldenummer: **02762219.0**

(22) Anmeldetag: **12.07.2002**

(51) Int Cl.:
***A61K 47/48*** *(2006.01)*

(86) Internationale Anmeldenummer:
**PCT/DE2002/002564**

(87) Internationale Veröffentlichungsnummer:
**WO 2003/006065 (23.01.2003 Gazette 2003/04)**

(54) **PNA-KONJUGAT ZUR THERAPIE VON MIT HIV IN ZUSAMMENHANG STEHENDEN ERKRANKUNGEN**

PNA CONJUGATE FOR THE TREATMENT OF DISEASES ASSOCIATED WITH HIV

CONJUGUE PNA POUR TRAITER DES AFFECTIONS ASSOCIEES AU VIH

(84) Benannte Vertragsstaaten:
**AT BE BG CH CY CZ DE DK EE ES FI FR GB GR IE IT LI LU MC NL PT SE SK TR**

(30) Priorität: **12.07.2001 DE 10133307**

(43) Veröffentlichungstag der Anmeldung:
**07.04.2004 Patentblatt 2004/15**

(73) Patentinhaber: **Deutsches Krebsforschungszentrum Stiftung des öffentlichen Rechts 69120 Heidelberg (DE)**

(72) Erfinder:
- **BRAUN, Klaus 69207 Sandhausen (DE)**
- **WALDECK, Waldemar 69514 Laudenbach (DE)**
- **PIPKORN, Rüdiger 69120 Heidelberg (DE)**
- **BRAUN, Isabell 35091 Cölbe-Bürgeln (DE)**
- **DEBUS, Jürgen 69121 Heidelberg (DE)**

(74) Vertreter: **Schüssler, Andrea Kanzlei Huber & Schüssler Truderinger Strasse 246 81825 München (DE)**

(56) Entgegenhaltungen:
**WO-A-01/05432** **WO-A-03/015708**
**WO-A-03/039438** **WO-A-03/040365**
**WO-A-03/047631**

- **PIPKORN, R. ET AL: "Peptide carrier for efficient drug transport into living cells" PEPTIDES: THE WAVE OF THE FUTURE, PROCEEDINGS OF THE SECOND INTERNATIONAL AND THE SEVENTEENTH AMERICAN PEPTIDE SYMPOSIUM, SAN DIEGO, CA, UNITED STATES, JUNE 9-14, 2001 (2001), 931-932.CODEN: 69DBAL; ISBN: 0-9715560-0-8, 9. Juni 2001 (2001-06-09), XP0008019046 EDITOR(S): LEBL, MICHAL; HOUGHTEN, RICHARD A. PUBLISHER: AMERICAN PEPTIDE SOCIETY, SAN DIEGO, CALIF.**
- **HALLBRINK M ET AL: "Cargo delivery kinetics of cell-penetrating peptides" BIOCHIMICA ET BIOPHYSICA ACTA - BIOMEMBRANES 01 DEC 2001 NETHERLANDS, Bd. 1515, Nr. 2, 1. Dezember 2001 (2001-12-01), Seiten 101-109, XP002248666 ISSN: 0005-2736**
- **BRAUN KLAUS ET AL: "A biological transporter for the delivery of peptide nucleic acids (PNAs) to the nuclear compartment of living cells." JOURNAL OF MOLECULAR BIOLOGY. 26 APR 2002, Bd. 318, Nr. 2, 26. April 2002 (2002-04-26), Seiten 237-243, XP002248667 ISSN: 0022-2836**
- **Pooga et al. (2001) Biomol. Engin. 17, 183-192.**
- **Donahue (1998) Nat. Med. 4, 181-186.**
- **Ray & Norden (2000) The Faseb Journal 14, 1041-1060.**
- **Cutrona et al. (2000) Nature Biotechnology 18, 300-303**
- **Lee et al. (1998) Biochemistry 37, 900-910**
- **McShan et al. (1992) JBC 267, 5712-5721**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 404 373 B1

## Beschreibung

Die vorliegende Erfindung betrifft Peptid-Nukleinsäure(PNA)-Konjugate, die zur Therapie von mit HIV in Zusammenhang stehenden Erkrankungen geeignet sind, wobei die Peptid-Nukleinsäure (PNA) die Genexpression von HIV hemmt.

Weltweit schreitet die Inzidenz des "human immunodeficiency virus (HIV) ungeachtet der bisherigen intensiven Forschungsbemühungen zur Entwicklung effektiver Behandlungsverfahren fort. HIV zählt zur lentiviralen Gruppe der Retroviren und ist eines der am intensivsten untersuchten Viren. Der HIVInfektionszyklus beginnt mit der Bindung viraler Partikel an die Zellmembran der Zielzellen mittels des viralen Hüllproteins gp120/gp41. Zunächst bindet das Virus an das CD4-Protein, danach folgt eine Anbindung an den obligatorischen Korezeptor, wobei es sich um ein Mitglied der Chemokin-Rezeptor-Familie handelt. Hauptziele der HIV-Infektion sind T-Helferzellen und Makrophagen. Dabei durchdringt der virale "Core"-Komplex die Zelle und das Virus integriert in das virale Genom in mehreren Schritten (reverse Transkription, Eintritt in den Zellkern, Integration in die Chromosomen der Wirtszellen als DNA-Doppelstrang). Ab diesem Zeitpunkt ist HIV permanenter Bestandteil des zellulären Genoms und kann als erworbene genetische Erkrankung betrachtet werden. HIV ist in der $CD4^+$-Zelle nicht in der Lage zu replizieren und benötigt zur "Initialzündung" seiner Promotoren in der regulatorischen Region (LTR) zelluläre Transkriptionsfaktoren zur Transkription früher regulatorischer mRNAs, die für Tat-, Rev- und Nef-Proteine kodieren. Das Transaktivatorprotein Tat ist in der frühen Phase der HIV-RNA-Synthese von besonderer Bedeutung. Die Tat-Proteinkonzentration korreliert direkt mit der Menge an HIV-RNA. Die Wechselwirkung zwischen Tat und TAR kann auch zu einer stark erhöhten frans-Aktivierung der viralen Genexpression führen, indem die Initiation der Transkription als auch die Elongation induziert werden.

Bisherige Therapieansätze, die eine kausale Behandlung zum Ziel hatten, brachten keinen entscheidenden Durchbruch bei der Bekämpfung von HIV-Infektionen. Medikamentöse Therapien waren bisher weder in der Lage, HIV-Infektionen zu stoppen noch hierdurch hervorgerufene Krankheiten zu helen. Immunologische Strategien, z.B. Impfungen, waren aufgrund der hohen Variabilität der Expressionsmuster der HIV-Virushüllproteine jedoch bisher auch nicht erfolgreich und erscheinen auch weiterhin wenig erfolgversprechend. Ein anderer theoretischer Therapieansatz basiert auf einer molekularen Virusinaktivierung, z.B. über Antisense-RNAs zur Blockierung vitaler Nukleinsäuren. Zwar bietet sich gerade bei HIV dieser Therapieansatz an, aber dieser scheint gerade wegen der transienten Kontrolle des HIV-Infektionszyklus und dem bisher kaum bekannten viralen Expressionsmuster sehr problematisch. Auch mit Ribozymen scheint eine HIV-Proliferationskontrolle nur sehr schwer realisierbar, da für eine solche Strategie spezielle geeignete CUG-Sequenzen des Virusgenoms identifiziert werden müssen (da die Ribozyme nur an diesem Sequenzmotiv schneiden), dies erscheint vor allem im Hinblick auf die sehr hohe Mutationsrate von HIV aber als äußerst schwierig.

Bei all den vorstehend diskutierten Vorgehensweisen taucht außerdem das Problem des wirksamen Einschleusens der Antisense- bzw. an den Zielort auf. Die bisher dazu verwendeten Vektoren, z.B. Adeno-assozierte Viren (AAV), weisen zahlreiche Nachteile auf. AAV sind kleine Parvoviren mit Einzelstrang-DNA, Ihr Potential liegt in ihrer Fähigkeit, teilende wie auch nichtteilende Zellen infizieren und sich ins Wirtsgenom integrieren zu können. Der Hauptnachteil liegt jedoch in der mangelnden Synthese ausreichender Mengen und mangelnder Stabilität als Vektor für hämatopoietische Zellen. Auf MLV ("murine leukemia virus") basierende Vektoren wurden ebenfalls in zahlreichen klinischen Studien getestet. Sie scheinen zwar untoxisch und theoretisch als möglicher Carrier für Antisense-Konstrukte geeignet zu sein, weisen jedoch den Nachteil auf, daß im Wirt nur sehr geringe Titer erreicht werden. Schließlich könnten noch auf LV (Lentiviren) basierende Vektoren in Frage kommen, allerdings ist bei diesen der große Nachteil, daß sie nur nicht-proliferierende Zellen infizieren können. Diese Eigenschaft würde zwar die Superinfektion HIVinfizierter Zellen erlauben, aufgrund ihres natürlichen viralen Tropismus sind sie jedoch trotzdem ungeeignet.

McShan et al. (1992) JBC 267, 5712-5721 offenbaren Oligonukleotide für eine Antigen-Strategie zur Inhibierung der Transkription von HIV in infizierter Zellen. Ferner offenbaren Lee et al. (1998) Biochemistry 37, 900-910 die Verwendung von Polyamid Nukleinsäuren (PNAs) zur inhibierung von der durch HIV-1 reversen Transkriptase vermittelten umgekehrten Transkription.

Die WO 01/05432 beschreibt ein Konjugat aus eine Transportprotein, einem Adressprotein und einem Wirkstoff, der eine PNA sein kann, zum Transport des Wirkstoffs in den Zellkern.

Somit liegt der vorliegenden Erfindung das technische Problem zugrunde, hatte bereitzustellen, die eine spezifische und effiziente auf einer Hemmung der Genexpression von HIV basierende Therapie erlauben.

Die Lösung dieses technischen Problems erfolgte durch die Bereitstellung der in den Patentansprüchen gekennzeichneten Ausführungsformen.

Von den Erfindern wurde zur Erzielung der Lösung des technischen Problems ein Konjugat entwickelt, das die folgenden Komponenten umfaßt:

- einen Transportvermittler für die Zellmembran ("P"),
- ein Adressprotein bzw. -peptid ("AP") für den Import in den Zellkern, und
- eine zu transportierende , mit mit dem Polypurintrakt des HIV Gens hybridisierbare Peptid-Nukleinsäure, die dessen Expression hemmt ("PNA")

Dieses modular aufgebaute Konjugat weist zwei entscheidende Vorteile auf:

(a) Mittels der Komponenten "P" und "AP" wird ein effizienter und gerichteter Transport der PNA zu dem Zielort und somit eine Gentherapie ermöglicht. Diese Komponenten erlauben nicht nur einen schnellen und effektiven Transport von Makromolekülen wie PNA durch Zellmembranen lebender Zellen ins Zytoplasma, sondern auch, nach zytoplasmatischer Aktivierung von Adresspeptidsequenzen, einen effizienten Transport in den Zellkern.

(b) Der Einsatz der protease- und nukleaseresistenten Peptid-Nukleinsäuren (PNA), bei denen es sich um Oligonukleotid-Derivate handelt, bei denen das Zuckerphosphat-Rückgrat bevorzugt durch Ethyl-Amin verbundene α-AminoEthyl-Glycin-Einheiten substituiert ist, erlaubt aufgrund ihrer physiko-chemischen Eigenschaften unter physiologischen Bedingungen eine stabile und effiziente Blockierung der Transkription des gewünschten Gens. Mit diesen PNAs wird eine auf dem Antisense-Prinzip basierende Anti-Gen-Strategie verfolgt, bei der jedoch nicht die mRNA sondern das Gen selbst, beispielsweise ein virales DNA-Intermediat oder die in genomische DNA des Wirts integrierte virale DNA, das Ziel ist. Dabei hybridisieren die PNAs über die Bildung einer Triple-Helix an die Ziel-DNA. Der Zielbereich ist die DNA des Polypurintraks des HIV-Gens , dessen Blockierung über die PNAs ebenfalls die Transkription hemmt.

Bezüglich Verfahren zur Herstellung der einzelnen Komponenten der Konjugate und zu deren Verknüpfung wird auf die deutsche Patentanmeldung Nr. 199 33 492.7 verwiesen. Die Synthese von PNAs ist dem Fachmann bekannt und z.B. auch in Nielsen et al., Science 254 (1991), 1497-1500, beschrieben.

Der Aufbau des erfindungsgemäßen Konjugats ist vorzugsweise:

**P - AP – (HIV-PNA)**

ganz bevorzugt mit einem Spacer ("SP"):

**P - AP - SP – (HIV-PNA)**

Den Transportvermittler für die Zellmembran (vorstehend mit "P" abgekürzt) stellt ein Peptid bzw. Protein dar, das die Plasmamembran überwinden kann. Die Länge dieses Peptids bzw. Proteins unterliegt keiner Beschränkung, solange es die obige Eigenschaft aufweist. Beispiele für "P" stammen vorzugsweise aus der Penetratin-Familie (Derossi et al., Trends Cell Biol. 8 (1988), S. 84-87) oder sind Transportan bzw. Teile davon (Pooga et al., The Faseb Journal 12 (1998), S. 68 ff.). wobei solche aus der Penetratin-Familie bevorzugt sind. Ein Beispiel für "P" stellt ein Penetratin mit der folgenden Sequenz dar:

$NH_2$-RQIKIWFQNRRMKWKK-

Hergestellt wird die ausgewählte "P"-Sequenz auf biologischem Weg (Reinigung natürlicher Transportvermittlerproteine oder Klonierung und Expression der Sequenz in einem eukaryotischen oder prokaryotischen Expressionssystem), bevorzugt aber auf synthetischem Weg, z.B. nach dem Merrifield-Verfahren (Merrifield, J. Am. Chem. Soc. 85 (1963), 2149).

Die Auswahl des Adressproteins bzw. -peptids (vorstehend mit "AP" abgekürzt) kann der Fachmann anhand der bekannten Aminosäuresequenzen für den Import in den Zellkern steuernde Peptide bzw. Polypeptide auswählen. Prinzipiell unterliegt die Länge dieses Adresspeptids bzw. -proteins keiner Beschränkung, solange es die Eigenschaft aufweist, einen zellkernspezifischen Transport zu gewährleisten. Für die Einbringung der PNAs werden im allgemeinen "AP" ausgewählt, die ein zellkernspezifisches Erkennungssignal enthalten und dadurch die PNAs in den Zellkern dirigieren. Grundsätzlich ist für den Transport in den Zellkern die reine Adressequenz ausreichend. Es können aber auch "AP" ausgewählt werden, die über eine zellkernspezifische Peptidasespaltstelle verfügen. Diese Spaltstelle liegt im günstigsten Fall innerhalb der Signalsequenz, kann aber auch an diese durch zusätzliche Aminosäuren angefügt werden, um nach Erreichen des Zellkerns das Abspalten der Adressequenz sicherzustellen. Hergestellt wird die ausgewählte "AP"-Sequenz auf biologischem (Reinigung natürlicher Transportvermittlerproteine oder Klonierung und Expression der Sequenz in einem eukaryontischen oder prokaryontischen Expressionssystem), bevorzugt aber auf synthetischem Weg, z.B. nach dem Merrifield-Verfahren (Merrifield, J. Am. Chem. Soc. 85 (1963), 2149). Beispiele für geeignete Adressproteine bzw. -peptide sind:

-Pro-Pro-Lys-Lys-Lys-Arg-Lys-Val; und
$H_3N^+$-Pro-Lys-Lys-Lys-Arg-Lys-Val-

(= "Nuclear localisation sequence" aus SV40-T-Antigen)

Weiter kann das Konjugat ggf. einen Spacer (vorstehend mit "SP" abgekürzt) enthalten, der sich vorzugsweise zwischen dem Adressprotein/-peptid und der zu transportierenden Peptid-Nukleinsäure (PNA) befindet. Er kann aber zusätzlich oder alternativ auch zwischen dem Transportvermittler und dem Adressprotein vorliegen. Der Spacer dient dazu, ggf. vorhandene sterische Wechselwirkungen zwischen den Komponenten aufzuheben bzw. günstig zu beeinflussen. Der Spacer kann beispielsweise ausgewählt sein aus: Glycin, Polylysin, Polyethylenglykol (PEG), Derivate der Poly-Methacrylsäure der Polyvinylpyrrolidon (PVP).

Zwischen dem Transportvermittler und dem Adressprotein/-peptid ist vorzugsweise eine Redoxspaltstelle, z.B. -Cystein-S-S-Cystein-O-N-H-. Die zwischen Transportvermittler und Adressprotein entstehende Bindung ist eine Redoxkopplung (schonende zellimmanente Verknüpfung mittels DMSO; Rietsch und Beckwith, Annu. Rev. Genet. 32 (1998), 163-84):

**Cystein-SH SH-Cystein ----> Cystin-S-S-Cystin**

Die Peptid-Nukleinsäure (PNA) erlaubt die Hemmung der Transkription vor für HIV essentiellen Genen, z.B. dadurch, daß sie mit einem Bereich des Polypyrintraks im Gen hybridisiert . Vorzugsweise weisen die Peptid-Nukleinsäuren eine Länge von mindestens 18 Basen auf, besonders bevorzugt sind Peptid-Nukleinsäuren mit einer Länge von mindestens 20 Basen. Die Peptid-Nukleinsäure kann ggf. auch markiert sein, z.B. radioaktiv (z.B. mit einem alpha-, beta- oder gamma-Strahler gekoppelt), mit einem Farbstoff, mit Biotin/Avidin usw.

Die Synthese der Konjugatbestandteile "P" und "AP" erfolgt vorzugsweise synthetisch nach dem Merrifield-Verfahren (Merrifield, J. Am. Chem. Soc. 85 (1963), 2149) Die Ankopplung der anderen Bestandteile (z.B. Spacer und/oder PNA) daran erfolgt durch kovalente chemische Bindung. Die Einfügung der Redoxspaltstelle zwischen "P" und "AP" erfolgt auf chemischen Wege durch die oben erwähnte Redoxkopplung. Auch zwischen einem ggf. vorhandenen Spacer und der PNA bzw. dem Adressprotein und der PNA liegt eine kovalente Bindung vor, bevorzugt eine Säureamid-Bindung. Mögliche Alternativen sind Ether- oder Ester-Bindungen, je nach den in der zu konjugierenden Substanz vorhandenen funktionellen Gruppe(n).

Die genomische Organisation von HIV-1 ist in Fig. 1 gezeigt.

Die für HIV-1 codierenden Sequenzen sind in Ratner et al., Nature 313, S. 277-284 (1985) veröffentlicht.

Die Verwendung von Pseudo-Isocytosin hat den Vorteil, daß eine pHunabhängige Hybridisierung möglich ist.

In den Fig. 9 bis 13 richten sich die PNAs gegen den Polypurin-Tract, zentralen DNA-Flap, Nef oder NCp7 (Vpr, Vpu) [s. Fig. 7]. Hierzu wurden von den Erfindern verschiedene Experimente durchgeführt, die in den Fig. 9 bis 13 gezeigt sind.

Bevorzugte PNAs gegen die vorstehend genannten Bereiche sind:

Sequenzen HIV-1 (es wird hier auf die Fig. 7-13 Bezug genommen)
Abkürzungen:
L = Linker
J = Pseudoisocytosin oder Cytosin

Für HIV :

c-PPT und 3'-PPT 4821-36 / 9116-31 (allg. Sequenz): **PNAI**
**(Polypurintrakt)**

N- TCC CCC CTT TTC TTT T- **L** -TTT T**J**T T

c-PPT target:
DNA (+) or RNA 4821-39

N-CA ATC CCC CCT TTT CTT T-**L**,TT T**J**T TT **<u>PNA Ia</u>**

1) nblaNLs+
2) nblaNLS-

(fortgesetzt)

| | | |
|---|---|---|
| | N- CA ATC CCC CCT TTT CTT T<br>(dieselbe Sequenz ohne Linker-Teil | **PNA Ib**<br>3) nbIbNLS+<br>4) nbIbNLS- |
| DNA (-) 4800-20 | | |
| | N- GTA TTC ATC CAC AAT TTT | **PNA II**<br>5) nbIIaNLs+<br>6) nbIIbNLS |
| DNA (+) 4800-20 | | |
| | N-AAA TTG TGG ATG AAT ACT | **PNA III**<br>7) nbIIIaNLS+<br>8) nbIIIbNLS |
| Flap:<br>DNA (-)<br>4861-80 | | **PNA IV** |
| | N-TAG TAG ACA TAA TAG CAA | **PNA IVa**<br>9) nbIVaNLS |
| Eine andere DNA (-) zwischen c-PPT and Tar, um die Länge des "Flap" zu verkürzen:<br>DNA(+)4841-60 | | |
| | N- TCC CCT GCA CTG TAC CCC | **PNA IVb**<br>- 10) nbVbNLS- |
| Nef: | | **PNA V** |
| DNA (-)<br>9095-9115 | | |
| | N-AGA TCT TAG CCA CTT TTT-C | **PNA Va**<br>11) nbVaNLS+ |
| 9136-56 | | |
| | N-GGC TAA TTC ACT CCC AAC-C | **PNA Vb**<br>12) nbVbNLS+ |
| | IN site (3') 9746-66:<br>N- TAG AGA TTT TCC ACA CTG | **PNA Vc**<br>13) nbVcNLS+ |
| Seq is gerichtet gegen den Start von gag: | | |
| | N- cac cca tct ctc tcc ttc (kein Linker) | **PNA VI**<br>14) nbVINLS- |
| splice acceptor site: | | |
| | N-**j**tt **j**tt-**L**-ttc ttc ctg cca tag | **PNA VII**<br>15) nbXNLS+<br>16) nbXNLS- |
| TAR | | |
| | N-cag gct caa atc tgg tct-**L**-t**j**t | **PNA VII**<br>17) nbXNLS- |
| NCp7: | | **PNA VIII** |
| | N-ATT ACT ACG TCT CTC CGT (nicht getestet) | 18)smVIIIaNLS+ |

(fortgesetzt)

| | |
|---|---|
| N-TAT CGG TTT TTA ACG TCC | 19)smVIIIaNLS+ |
| N T TTT **JJ**T- linker -TCC TTT TCC CCG ACA ACC | 20)smVIIIc NLS+ |

| | |
|---|---|
| Random Sequenz als Kontrolle : | **PNA IX** |
| N-CAT ACT TGA CTC GTT ATC-C | 21) IX NLS- |
| N-CAT ACT TGA CTC GTT ATC-C | 22) IX NLS+ |

BDV PNA: (diese Sequenz wurde für das BDV spreading getestet; Fig. 8) N- TCC CTA CGC CGC CTT CTC-C terminus

Die vorliegende Erfindung betrifft schließlich auch ein erfindungsgemäßes Konjugat, gegebenenfalls zusammen mit einem geeigneten Träger, enthaltendes Arzneimittel sowie dessen Verwendung zur HIV-Therapie. Als geeignet hat sich dabei insbesondere die parenterale oder intravenöse Applikation herausgestellt.

Die Erfindung wird weiter anhand der Figuren beschrieben, welche zeigen:

- Fig. 1: genomische Organisation von HIV-1
- Fig. 2: Sequenzen der LTR-Region von HIV-1
- Fig. 3: Plasmid-Karte von pEGFP-C3
- Fig. 4: Virale Angriffsorte der Pseudo-Isocytosin enthaltenden PNAs
- Fig. 5: CLSM-Aufnahmen (Nicht aktivierte Kontrolle in HeLa)
- Fig. 6: CLSM-Aufnahmen (Nach Aktivierung in HeLa)
- Fig. 7: PNA-"Targets"
- Fig. 8: Untersuchung von BDV (Borna disease virus; retrovirus) anstatt von HIV zum Beweis, daß auch andere Retroviren durch PNA-Konstrukte inhibierbar sind
- Fig. 9: Effizienz von PNA in der frühen Phase viraler Infektion Der biologische Effekt wurde gemessen durch Transaktivierung: eines Reporter-Genkonstrukts (LacZ)
- Fig. 10: Ergebnisse des Reporter-Genassays nach Behandlung mit verschiedenen PNAs. Signifikante Ergebnisse wurden mit PNAs gegen cPPT (Polypurin-Tract) gesehen
- Fig. 11: Verwendung von PNAs in der frühen und späten Phase viraler Infektion; Testung durch ELISA gegen p24 Virenprotein. Verschiedene PNAs gegen cPPT/Flap/Nef/gag/Random waren fahig, das virale p24 drastisch zu reduzieren
- Fig. 12: Reporter-Genassay mit anti-FLAP PNA kombiniert mit anderen PNAs. Die Kombination von PNA[IV b] + PNA [IIINLS] + PNA IV b] (500 nM Konzentration) resultierte in optimaler β-Gal Reduktion.
- Fig. 13: Chronisch HIV infizierte Zellen wurden nach PNA Behandlung durch viralen p24 ELISA untersucht (72 und 144 Std. nach FNA-Applikation). Der beste Effekt wurde bei cPPT (Polypurin-Trakt) durch PNA [IINLS] erhalten,

Die Erfindung wird weiter anhand der nachfolgenden Beispiele beschrieben.

**Beispiel 1** (nicht Gegenstand der Erfindung)

Der HIV-1 "long terminal repeat" (LTR) codiert für den Transkriptionspromotor. Sequenzanalysen belegen die Existenz einer einzigen LTR Enhancer-Promotor Konfiguration für sämtliche bisher untersuchten HIV-1 Subtypen. Transkriptionsstudien mit EGF-Reporter-Konstrukten zeigen seine Funktionalität.

<u>LTR-EGF-Konstrukt:</u>

Basierend auf Biocomputing Daten wurden mit dem HUSAR-Progamm des DKFZ, dem SRS (Sequence Retrieval System) und dem multiplen Alignement-Algorithmus (MALIGN) repräsentativ die Sequenz AC S72615, die den HIV-Subtyp HIV4B6 betrifft, ausgewählt (Shiramizu et al., Cancer Res. 54, S. 2069-2072 (1994)).

HIV4B6-LTR:

CCAATAAAGG AGAAAACAAC TGCTTGTTAC ACCC$_{(-18)}$TATAAG
CCAGCATAAA GC$_{(+1)}$ATG GA

Das Ase | (8/-52)-Nhe | (64/+1)-LTR-Fragment wurde gemäß dem bekannten Phosphoramidit-Verfahren synthetisiert und in eine pEGFP-C3/Variante (ohne PCMV) (Fig. 3) (Fa. Clontech, Heidelberg) einkloniert.

Einkloniertes Fragment:

3´-$_{(-52)}$CCAATAAAGG AGAAAACAAC TGCCTTGTTAC ACCC$_{(-18)}$TATAAG
CCAGCATAAA GC-5´

Als nächstes wurde die Plasmid-DNA in E.coli in LB-Amp-Kulturmedium unter Ampicillin-Selektionsdruck vermehrt. Die Isolation und Aufbereitung der Plasmid-DNA wurde mit dem Mini-Prep-DNA Kit (Fa. Clontech) gemäß Herstellerangaben durchgeführt.

<u>Zellkultur und Transfektions-Assay:</u>

Verwendet wurde die humane Cervix-Karzinom Suspensions-Zellinie HeLa-S (Tumorbank DKFZ). Die Zellen wurden in MEM"Joklik" (Minimal Essential Medium; Fa. Sigma) mit 10% FCS (Fa. Sigma), Glutamin (Fa. Gibco) in einer $CO_2$ Atmosphäre bei 37°C kultiviert.

Es wurde folgendes "Shuttle-Konstrukt" hergestellt:

RQQIKIWFQNRRMKWKK-SS-PKKKEKV-GG-GK-TTA TTT CCT CCT TTG TTG
→TTA TTT CCT (= zur Stabilisierung)

Dieses Konstrukt wurde nach dem Protokoll von Britten und Kohne (Science, Vol. 161, No. 3841, S. 529-540, 1986) in die HeLa-Zellen transfiziert. Die Transfektion der HeLa-S erfolgt durch direkte Zugabe des "Shuttle-Konstrukts" in das Kulturmedium, in einer Konzentration von 100 pM, über 3 Stunden bei 37°C unter Inkubation in 5% CO2-Atmosphäre. Danach erfolgte die Hybridisierung mit dem LTR-EGF-Konstrukt anlog dem oben erwähnten Britten und Kohne-Protokoll. Dabei entsteht folgender "Komplex":

......GG-GK-TTA TTT CCT CTT TTG TTG
AAT AAA GGA GAA AAC AAC
→TTA TTT CCT

Die Kultivierung der HeLa-S Zellen erfolgt in 8-Kammer Glasplatten in Petrischalen unter 5% CO2 bei 37°C über 48 Stunden.

Zur Aktivierung des Plasmids ist es notwendig, das "Shuttle-System" von der Plasmid-DNA zu trennen. Hierzu werden die Platten über 1 Minute auf 45°C im Wasserbad erhitzt. Nach Mediumwechsel werden die HeLa-S-Zellen weiterkultiviert. Die Transkriptionsbestimmung von GFP unter LTR-Kontrolle erfolgt mittels Fluoreszenz-Reader-Analyse nach 48, 72 und 96 Stunden (Anregung: 488 nm; Emission: 520 nm). Die Lokalisierung des GFP mittels konfokaler Laser Scanning Mikroskopie (CLSM) erfolgt analog, jedoch in 8-Kammer-Glasplatten in Petrischalen unter 5% $CO_2$ bei 37°C über 48, 72 und 96 Stunden (Anregung: 488 nm; Emission: 520 nm). Die Ergebnisse sind in Fig. 5 und 6 gezeigt.

**Beispiel 2**

Hier ist eine Methode beschrieben, die erlaubt, einen PNA Effekt in einer frühen Infektionsphase von HIV-1 zu bestimmen. Es wird hier Bezug auf die Fig. 9-11 genommen.

Als Modell wurden HeLa Zellen verwendet, die mit einem stabil exprimierten LacZ Reportergen transfiziert wurden. Durchgeführt wurde der Test in einem 96-Wellplate. HeLa Zellen werden in RPMI Medium suspendiert und ausplattiert. Die Zellzahl war 20000 Zellen pro Well. Das Pipettiervolumen beträgt 100 μl/Well. Getestet wurden 18 unterschiedliche PNA-Sequenzen (s. Fig. 10) plus eine Kontrolle (X = weitere virale Kontrolle (z.B. BDV)). Die PNA-Konstrukt-Konzentration waren 10 pM, 100 pM und 1 nM. Die PNA-Konstrukte waren analog Beispiel 1 hergestellt. Nach einer Stunde erfolgte dann die virale Infektion. Der virale Effekt auf den LTR Promotor wurde über die LacZ Aktivität mittels Photometer ermittelt.

## Patentansprüche

**1.** Konjugat zur Vermittlung eines zeflkernspezifischen Transports einer Peptid-Nukleinsäure (PNA), die gegen den Polypurintrakt des HIV-Gens gerichtet ist, wobei das Konjugat die folgenden Komponenten aufweist:

- einen Transportvermittler für die Zellmembran,
- ein Adressprotein bzw. -peptid für den Import in den Zellkern, und
- eine zu transportierende, mit der DNA Sequenz des Polypurintrakts des HIV-Gens hybridisierbare Peptid-Nukleinsäure (PNA).

**2.** Konjugat nach Anspruch 1, wobei der Transportvermittler ein oder Protein ist, das die Plasmamembran überwinden kann.

**3.** Konjugat nach Anspruch 1 oder 2, wobei der Transportvermittler aus der Penetratin-Familie stammt oder Transportan oder Teile davon ist.

**4.** Konjugat nach Anspruch 3, wobei eines der Penetratine folgende Sequenz hat:

$NH_2$-RQIKIWFQNRRMKWKK-

**5.** Konjugat nach einem der Ansprüche 1 bis 4, wobei das Adressprotein bzw. - peptid ausgewählt ist aus:

-Pro-Pro-Lys-Lys-Lys-Arg-Lys-Val; und
$H_3N^+$-Pro-Lys-Lys-Lys-Arg-Lys-Val-

(= Nuclear localisation sequence aus SV40-T-Antigen)

**6.** Konjugat nach einem der Ansprüche 1 bis 5, wobei das Konjugat folgenden Aufbau hat:

Transportvermittler - Adressprotein - Peptid-Nukleinsäure (PNA)

**7.** Konjugat nach einem der Ansprüche 1 bis 6, wobei ggf. weiter ein Spacer vorhanden ist.

**8.** Konjugat nach Anspruch 7, wobei sich der Spacer zwischen dem Adressprotein und der Peptid-Nukleinsäure (PNA) befindet

**9.** Konjugat nach Anspruch 7 oder 8, wobei der Spacer Polylysin, Polyethylenglykol oder Polyvinylpyrrolidon ist.

**10.** Konjugat nach einem der Ansprüche 1 bis 9, wobei die Peptid-Nukleinsäure eine Sequenz ausgewählt aus folgender Gruppe umfasst:-

CAATCCCCCCTTTTCTTT-L-TTTJTTT:
CAATCCCCCCTTTTCTTT;
GTATTCATCCACAATTTT; und
AAATTGTGGATGAATACT,

Worin: L = Linker, und J = Pseudoisocytosin oder Cytosin.

**11.** Arzneimittel, ein Konjugat nach einem der Ansprüche 1 bis 10 enthaltend.

12. Arzneimittel nach Anspruch 11, das eine Kombination von Konjugaten mit drei verschiedenen Peptid-Nukleinsäuren umfasst, wobei die Peptid-Nukleinsäuren TCCCCTGCACTGTACCCC, AAATTGTGGATGAATACT und GGCTAATTCACTCCCAACC sind.

13. Verwendung eines Konjugats nach einem der Ansprüche 1 bis 10 oder des Arzneimittels nach Anspruch 12 zur Herstellung eines Medikamentes zur Verwendung in der HIV-Therapie.

## Claims

1. A conjugate for mediating a cell nucleus-specific transport of a peptide nucleic acid (PNA) which is directed against the polypurine tract of the HIV gene, wherein the conjugate comprises the following components:

- a transport mediator for the cell membrane,
- an address protein or peptide for the import into the cell nucleus, and
- a peptide nucleic acid (PNA) which is to be transported and can be hybridized with the DNA sequence of the polypurine tract of the HIV gene.

2. The conjugate according to claim 1, wherein the transport mediator is a peptide or protein which can overcome the plasma membrane.

3. The conjugate according to claim 1 or 2, wherein the transport mediator originates from the penetratin family or is transportan or parts thereof.

4. The conjugate according to claim 3, wherein one of the penetratins has the following sequence:

$NH_2$-RQIKIWFQNRRMKWKK-

5. The conjugate according to any of claims 1 to 4, wherein the address protein or peptide is selected from:

-Pro-Pro-Lys-Lys-Lys-Arg-Lys-Val; and
$H_3N^+$-Pro-Lys-Lys-Lys-Arg-Lys-Val-

(= nuclear localization sequence from SV40-T antigen).

6. The conjugate according to any of claims 1 to 5, wherein the conjugate has the following structure:

transport mediator - address protein - peptide nucleic acid (PNA).

7. The conjugate according to any of claims 1 to 6, wherein a spacer is also present, where appropriate.

8. The conjugate according to claim 7, wherein the spacer is located between the address protein and the peptide nucleic acid (PNA).

9. The conjugate according to claim 7 or 8, wherein the spacer is polylysine, polyethylene glycol or polyvinyl pyrrolidone.

10. The conjugate according to any of claims 1 to 9, wherein the peptide nucleic acid is a sequence selected from the following group:

CAATCCCCCCTTTTCTTT-L-TTTJTTT;
CAATCCCCCCTTTTCTTT
GTATTCATCCACAATTTT; and
AAATTGTGGATGAATACT,

wherein L = linker, and J= pseudoisocytosine or cytosine

11. A medicament comprising a conjugate according to any of claims 1 to 10.

**12.** The medicament according to claim 11 comprising a combination of three different peptide nucleic acids, wherein the peptide nucleic acids are TCCCCTGCACTGTACCCC, AAATTGTGGATGAATACT and GGCTAATTCACTCCCAACC:

**13.** A use of a conjugate according to any of claims 1 to 10 or of the medicament according to claim 12 for the preparation of a medicament for the HIV therapy.

**Revendications**

**1.** Conjugué pour déterminer un transport spécifique au noyau de cellule d'un acide nucléique de peptide (PNA) qui est dirigé contre le tractus de polypurine du gène VIH, le conjugué présentant les composants suivants :

- un agent transporteur de la membrane de cellule,
- une protéine ou un peptide d'adresse pour l'importation dans le noyau de cellule,
et
- un acide nucléique de peptide (PNA) à transporter pouvant être hybridé avec la séquence d'ADN du tractus de polypurine du gène VIH.

**2.** Conjugué selon la revendication 1, l'agent transporteur étant un peptide ou une protéine pouvant vaincre la membrane de plasma.

**3.** Conjugué selon la revendication 1 ou 2, l'agent transporteur étant issu de la famille des pénétratines ou étant transportan ou des parties de ce dernier.

**4.** Conjugué selon la revendication 3, l'une des pénétratines ayant la séquence suivante :

NH2-RQIKIWFQNRRMKWKK-

**5.** Conjugué selon l'une des revendications 1 à 4, la protéine ou le peptide d'adresse étant sélectionné parmi :

-Pro-Pro-Lys-Lys-Lys-Arg-Lys-Val; et
$H_3N^+$-Pro-Lys-Lys-Lys-Arg-Lys-Val-

(= séquence de localisation nucléaire d'antigène SV40-T)

**6.** Conjugué selon l'une des revendications 1 à 5, le conjugué ayant la construction suivante :

agent transporteur - protéine d'adresse - acide nucléique de peptide (PNA)

**7.** Conjugué selon l'une des revendications 1 à 6, un espaceur étant éventuellement par ailleurs présent.

**8.** Conjugué selon la revendication 7, l'espaceur se trouvant entre la protéine d'adresse et l'acide nucléique de peptide (PNA) .

**9.** Conjugué selon la revendication 7 ou 8, l'espaceur étant polylysine, polyéthylène-glycol ou polyvinylpyrrolidone.

**10.** Conjugué selon l'une des revendications 1 à 9, l'acide nucléique de peptide comprenant une séquence sélectionnée parmi le groupe suivant :

CAATCCCCCCTTTTCTTT-L-TTTJTTT ;
CAATCCCCCCTTTTCTTT ;
GTATTCATCCACAATTTT ; et
AAATTGTGGATGAATACT;

dans quel L=lieur peptidique, et J=pseudoisocytosine ou cytosine.

**11.** Médicament contenant un conjugué selon l'une des revendications 1 à 10.

12. Médicament selon la revendication 11, comprenant une combinaison de conjugués avec trois acides nucléiques de peptide différents, les acides nucléiques de peptide étant TCCCCTGCACTGTACCCC, AAATTGTGGATGAATACT et GGCTAATTCACTCCCAACC.

13. Utilisation d'un conjugué selon l'une des revendications 1 à 10 ou du médicament selon la revendication 12 pour traiter le VIH.

# HIV-1 LTR-Kontrollregion, Genom, Genlocus, Protein und Funktionen

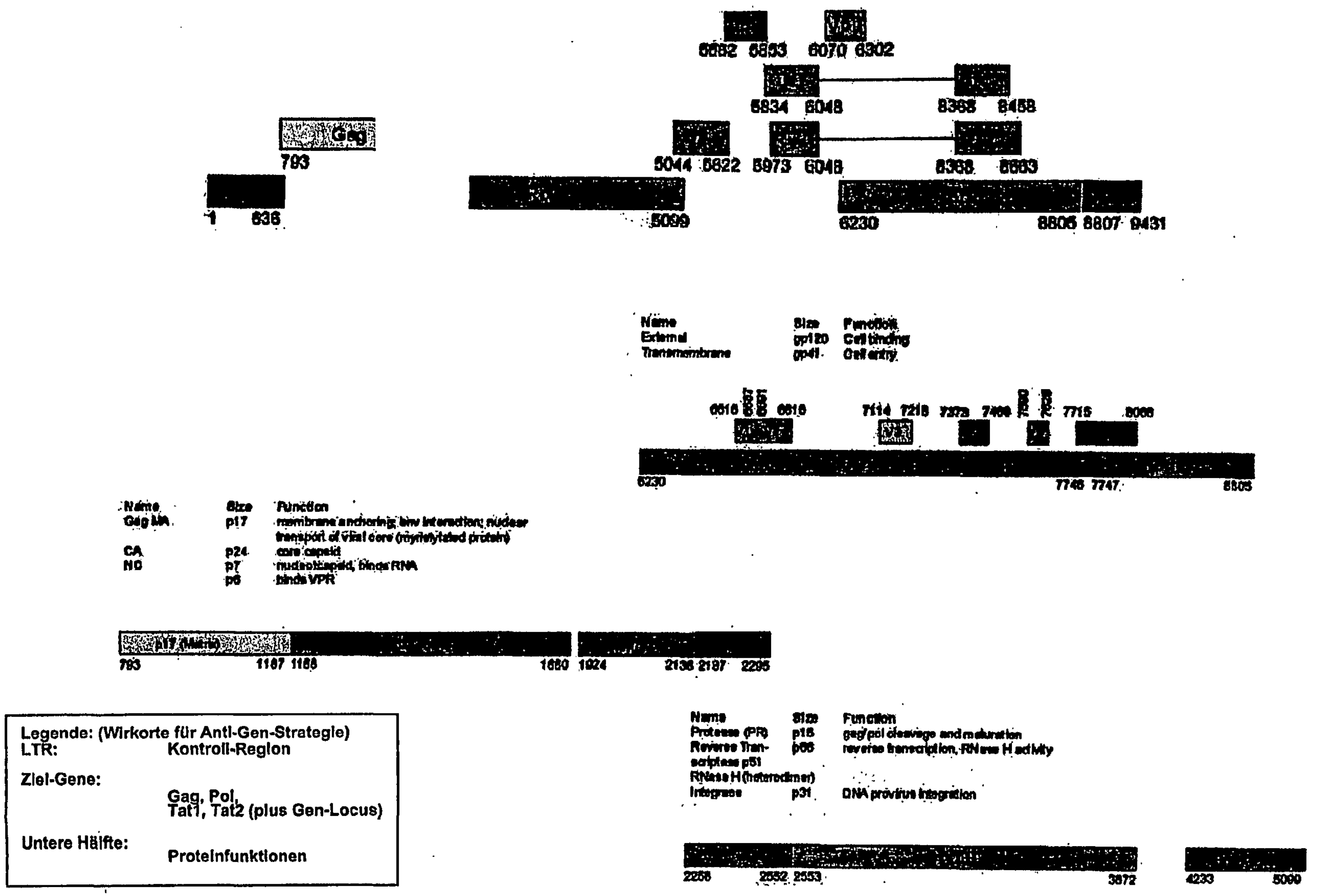


Fig. 1

```
LOCUS       HIVHXB2CG    9719 bp ss-
DEFINITION  Human immunodeficiency virus type 1 (HXB2), complete genome;
            HIV1/HTLV-III/LAV reference genome.
ACCESSION   K03455 M38432
VERSION     K03455.1  GI:1906382
KEYWORDS    TAR protein; acquired immune deficiency syndrome; complete enome;env
protein; gag protein; long terminal repeat (LTR); pol protein;polyprotein;
proviral gene; reverse transcriptase; transactivator.
SOURCE      Human immunodeficiency virus type 1.
FEATURES             Location/Qualifiers
     source          1..9719
                     /organism="Human immunodeficiency virus type 1"
                     /db_xref="taxon:11676"
                     /note="HTLV-III/LAV"
     LTR             1..634
                     /note="5' LTR"
     repeat_region   454..551
                     /note="R repeat 5' copy"
     mRNA            455..9635
                     /note="HXB2 genomic mRNA"
     prim_transcript 455..9635
                     /note="tat, trs, 27K subgenomic mRNA"
     intron          744..5777
                     /note="tat, trs, 27K mRNA intron 1"
     CDS             790..2292

BASE COUNT     3411 a   1772 c   2373 g   2163 t
ORIGIN
     TGGAAGGGCT AATTCACTCC CAACGAAGAC AAGATATCCT TGATCTGTGG ATCTACCACA      60
     CACAAGGCTA CTTCCCTGAT TAGCAGAACT ACACACCAGG GCCAGGGATC AGATATCCAC     120
     TGACCTTTGG ATGGTGCTAC AAGCTAGTAC CAGTTGAGCC AGAGAAGTTA GAAGAAGCCA     180
     ACAAAGGAGA GAACACCAGC TTGTTACACC CTGTGAGCCT GCATGGAATG GATGACCCGG     240
     AGAGAGAAGT GTTAGAGTGG AGGTTTGACA GCCGCCTAGC ATTTCATCAC ATGGCCCGAG     300
     AGCTGCATCC GGAGTACTTC AAGAACTGCT GACATCGAGC TTGCTACAAG GGACTTTCCG     360
     CTGGGGACTT TCCAGGGAGG CGTGGCCTGG GCGGGACTGG GGAGTGGCGA GCCCTCAGAT     420
     CCTGCATATA AGCAGCTGCT TTTTGCCTGT ACTGGGTCTC TCTGGTTAGA CCAGATCTGA     480
     GCCTGGGAGC TCTCTGGCTA ACTAGGGAAC CCACTGCTTA AGCCTCAATA AAGCTTGCCT     540
     TGAGTGCTTC AAGTAGTGTG TGCCCGTCTG TTGTGTGACT CTGGTAACTA GAGATCCCTC     600
     AGACCCTTTT AGTCAGTGTG GAAAATCTCT AGCAGTGGCG CCCGAACAGG GACCTGAAAG     660
     CGAAAGGGAA ACCAGAGGAG CTCTCTCGAC GCAGGACTCG GCTTGCTGAA GCGCGCACGG     720
     CAAGAGGCGA GGGGCGGCGA CTGGTGAGTA CGCCAAAAAT TTTGACTAGC GGAGGCTAGA     780
     AGGAGAGAGA TGGGTGCGAG AGCGTCAGTA TTAAGCGGGG GAGAATTAGA TCGATGGGAA     840
     AAAATTCGGT TAAGGCCAGG GGGAAAGAAA AAATATAAAT TAAAACATAT AGTATGGGCA     900
     AGCAGGGAGC TAGAACGATT CGCAGTTAAT CCTGGCCTGT TAGAAACATC AGAAGGCTGT     960
     AGACAAATAC TGGGACAGCT ACAACCATCC CTTCAGACAG GATCAGAAGA ACTTAGATCA    1020
     TTATATAATA CAGTAGCAAC CCTCTATTGT GTGCATCAAA GGATAGAGAT AAAAGACACC    1080
     AAGGAAGCTT TAGACAAGAT AGAGGAAGAG CAAAACAAAA GTAAGAAAAA AGCACAGCAA    1140
     GCAGCAGCTG ACACAGGACA CAGCAATCAG GTCAGCCAAA ATTACCCTAT AGTGCAGAAC    1200
     ATCCAGGGGC AAATGGTACA TCAGGCCATA TCACCTAGAA CTTTAAATGC ATGGGTAAAA    1260
     GTAGTAGAAG AGAAGGCTTT CAGCCCAGAA GTGATACCCA TGTTTTCAGC ATTATCAGAA    1320
     GGAGCCACCC CACAAGATTT AAACACCATG CTAAACACAG TGGGGGGACA TCAAGCAGCC    1380
     ATGCAAATGT TAAAAGAGAC CATCAATGAG GAAGCTGCAG AATGGGATAG AGTGCATCCA    1440
     GTGCATGCAG GGCCTATTGC ACCAGGCCAG ATGAGAGAAC CAAGGGGAAG TGACATAGCA    1500
     GGAACTACTA GTACCCTTCA GGAACAAATA GGATGGATGA CAAATAATCC ACCTATCCCA    1560
     GTAGGAGAAA TTTATAAAAG ATGGATAATC CTGGGATTAA ATAAAATAGT AAGAATGTAT    1620
     AGCCCTACCA GCATTCTGGA CATAAGACAA GGACCAAAGG AACCCTTTAG AGACTATGTA    1680
     GACCGGTTCT ATAAAACTCT AAGAGCCGAG CAAGCTTCAC AGGAGGTAAA AAATTGGATG    1740
     ACAGAAACCT TGTTGGTCCA AAATGCGAAC CCAGATTGTA AGACTATTTT AAAAGCATTG    1800
     GGACCAGCGG CTACACTAGA AGAAATGATG ACAGCATGTC AGGGAGTAGG AGGACCCGGC    1860
     CATAAGGCAA GAGTTTTGGC TGAAGCAATG AGCCAAGTAA CAAATTCAGC TACCATAATG    1920
     ATGCAGAGAG GCAATTTTAG GAACCAAAGA AAGATTGTTA AGTGTTTCAA TTGTGGCAAA    1980
     GAAGGGCACA CAGCCAGAAA TTGCAGGGCC CCTAGGAAAA AGGGCTGTTG GAAATGTGGA    2040
     AAGGAAGGAC ACCAAATGAA AGATTGTACT GAGAGACAGG CTAATTTTTT AGGGAAGATC    2100
     TGGCCTTCCT ACAAGGGAAG GCCAGGGAAT TTTCTTCAGA GCAGACCAGA GCCAACAGCC    2160
     CCACCAGAAG AGAGCTTCAG GTCTGGGGTA GAGACAACAA CTCCCCCTCA GAAGCAGGAG    2220
     CCGATAGACA AGGAACTGTA TCCTTTAACT TCCCTCAGGT CACTCTTTGG CAACGACCCC    2280
     TCGTCACAAT AAAGATAGGG GGGCAACTAA AGGAAGCTCT ATTAGATACA GGAGCAGATG    2340
     ATACAGTATT AGAAGAAATG AGTTTGCCAG GAAGATGGAA ACCAAAAATG ATAGGGGGAA    2400
     TTGGAGGTTT TATCAAAGTA AGACAGTATG ATCAGATACT CATAGAAATC TGTGGACATA    2460
```

Fig. 2

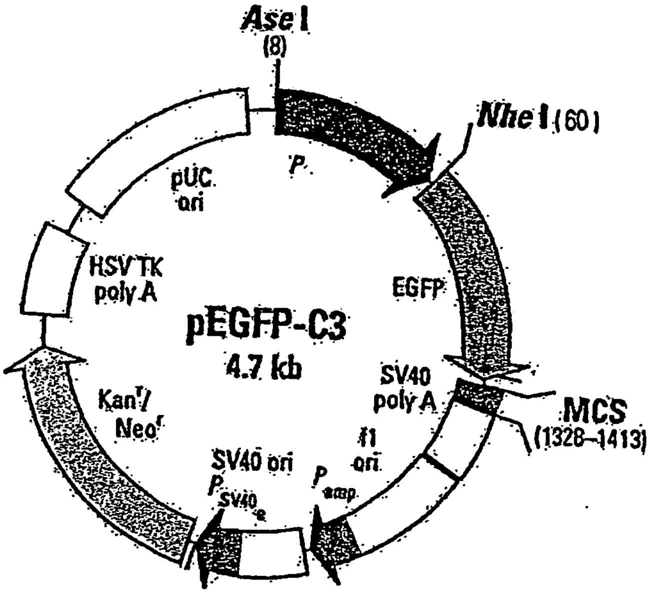
Ase I
(8)
Nhe I (60)
P
pUC ori
HSV TK poly A
EGFP
pEGFP-C3
4.7 kb
SV40 poly A
MCS
(1328–1413)
Kan^r/ Neo^r
f1 ori
SV40 ori
P SV40 e
P amp

Fig. 3

```
                  .   :   .   :   .   :   .   :   .   :   .   :
HIUU70276                 G-CCTGGCCATAAAGCAAGAATTTTGGCTGAGGCAATGAGCC
HIUU70291    CATGTCGGGGAGTGGGAGGACCTAGCCACAAAGCCAGAGTGTTGGCTGAGGCAATGAGCC
HIUU70281    CATGTCASGGAGTGGG-GGACCCGGCCATAAAGCAAGAGTTTTGGCTGAAGCAATGAGCC
HIVU70290                              CATAANGCAAGAGTTTTGGCTGAAGCAATGAGCC
HIUU70292              AGTGGGAGGMCCCGGCCAMAAAGCAAGGGTTTTGGCGGAAGCAATGAGCC
HIVU70292              AGTGGGAGGMCCCGGCCAMAAAGCAAGGGTTTTGGCGGAAGCAATGAGCC

                                    PNA (1)
                  .   :   .   :   .   :   .   :   .   :   .   :
HIUU70276    AGGTAACAAATACRGCTG---TAATGATGCAGCGAAACAACTTTAAGGGT--CAAAGAAA
HIUU70291    AAGCAAATAATACA---AACATAATGATGCAGAGAAACAACTTTAAAGGC-CCTAA-AAG
HIUU70281    AAGTAACACCACCAGCTAACATAATGATGCAGAGAGGCAATTTTA--GGAACCAAAGAAA
HIVU70290    AAGTAACACAACCAGCTACCATAATGATGCAGAGAGGCAATTTTA--GGAACCAAAGAAA
HIUU70292    AAGTAACAAATTCACCTGCCATAATGATGCAGAGAGGCAATTTTA--GGAACCAAAGAAA
HIVU70292    AAGTAACAAATTCACCTGCCATAATGATGCAGAGAGGCAATTTTA--GGAACCAAAGAAA

                                                         PNA (2)
                  .   :   .   :   .   :   .   :   .   :   .   :
HIUU70276    AATTATTAAATGTTTCAACTGTGGCANGGAGGGACACYTAGCAAAAAATTGCAGGGCCCC
HIUU70291    AATTATTAAATGTTTCAACTGTGGCAAGGAAGGGCACTTAGCCAGAAATTGCAGGGCCCC
HIUU70281    GACTGTTAAGTGTTTCAATTGTNNNDAAGAAGGGCAYATAGCCAAAAATTGCAGGGCCCC
HIVU70290    GACTGTTAAGTGTTTCAATTGBBBVAAAGAAGGGCACATAGCCAAAAATTGCAGGGCCCC
HIUU70292    AACTGTTAAGTGTTTCAATTGTGGCAAAGAAGGGCACATAGCCAAAAATTGCAGGGCCCC
HIVU70292    AACTGTTAAGTGTTTCAATTGTGGCAAAGAAGGGCACATAGCCAAAAATTGCAGGGCCCC

                     PNA (3)
                  .   :   .   :   .   :   .   :   .   :   .   :
HIUU70276    TAGGDDGDDGGGTTGTTGGAAATGT---AA
HIUU70291    TAGGAAAAAAAGGCTGTTGGAAATGTGGAAAGGAAGGAC
HIUU70281    TAGGAADAAGGGCTGTTGGAAATGT
HIVU70290    TAGGAAAAAGGGCTGTTGGAAATGTGGTAGGGAAGGACAC
HIUU70292    TAGGAAAAGGGGCTGTTGGAAATGTGGHAAGGAAGGAM
HIVU70292    TAGGAAAAGGGGCTGTTGGAAATGTGGHAAGGAAGGAM
```

(1) ATTAC*TAC*GTC*TC*TC*C*GTT

(2) TATC*GGTTTTTAAC*GTC*C*C*

(3) TC*C*TTTTC*C*C*C*GAC*AAC*C*TTTAC*

Fig. 4

# LTR-EGFP Reportergen

Nicht aktivierte Kontrolle in HeLa
Excit.: 488 nm
Em.: 520 nm
Nukleus: DAPI Färbung
CLSM

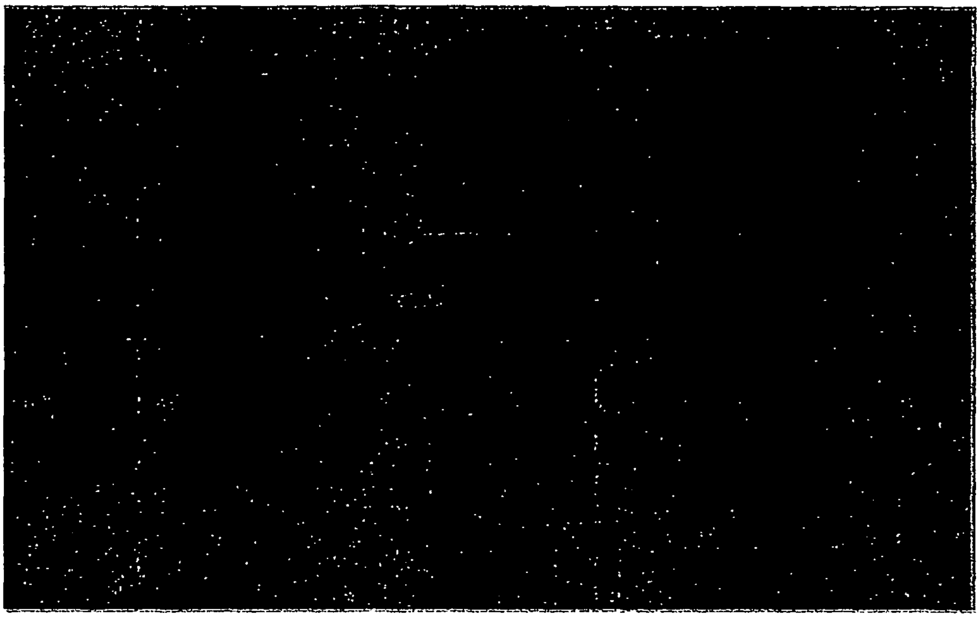

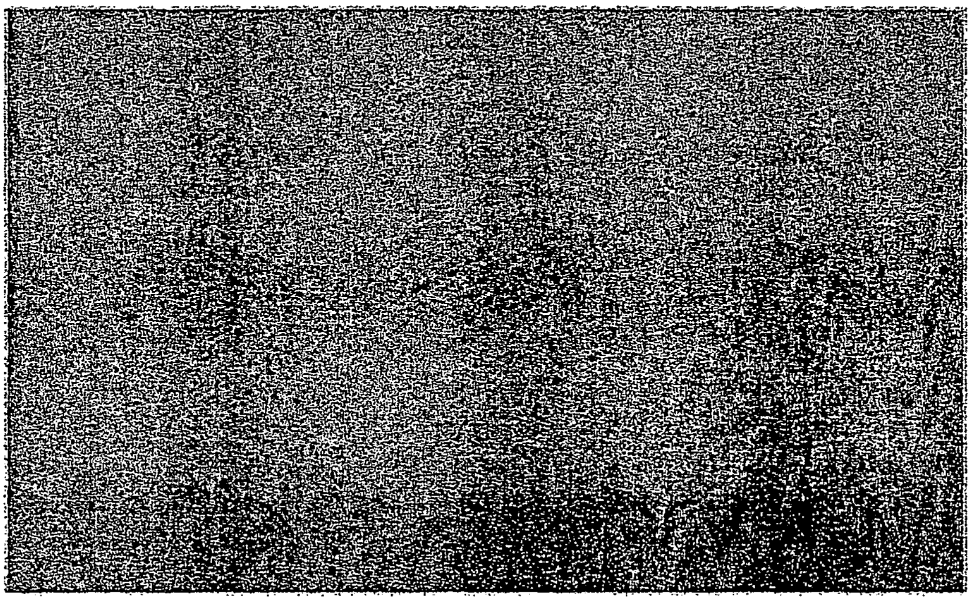

Fig. 5

# LTR-EGFP Reportergen

Nach Aktivierung in HeLa
Excit.: 488 nm
Em.: 520 nm
Nukleus: DAPI Färbung
CLSM

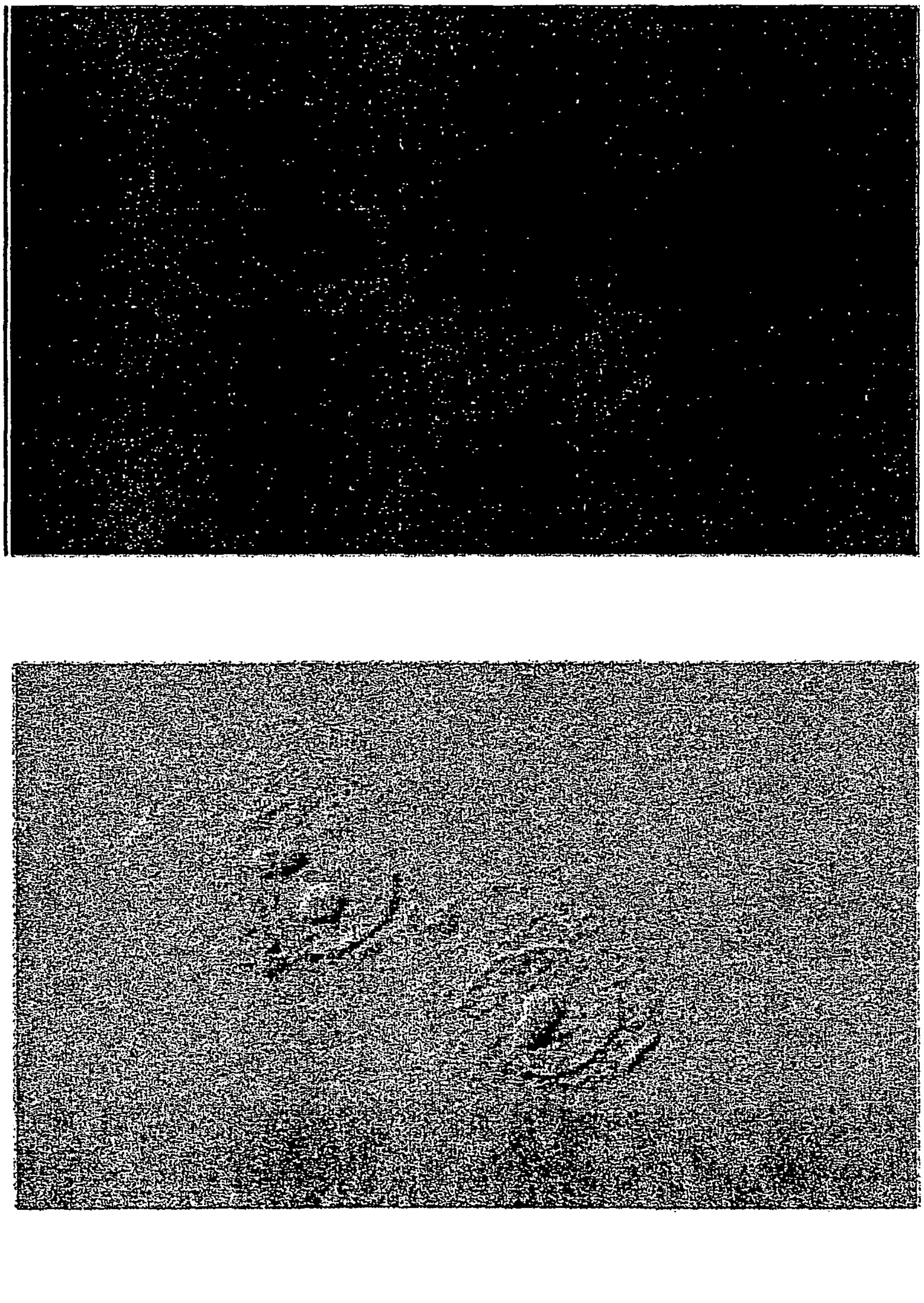

Fig. 6

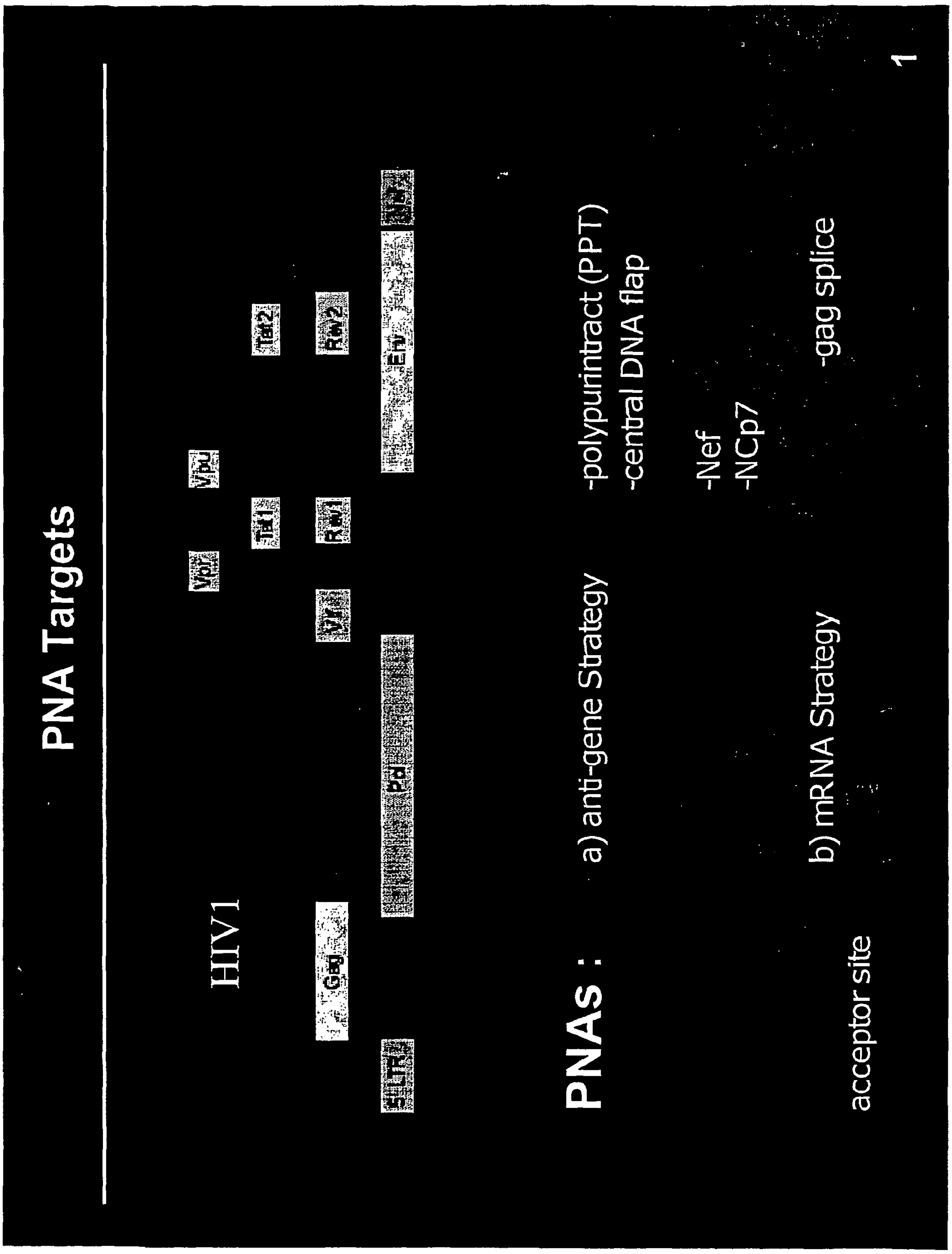
PNA Targets
HIV1
Gag
Pol
Tat1
Rev1
Vpu
Tat2
Rev2
Env
PNAs :
a) anti-gene Strategy
-polypurintract (PPT)
-central DNA flap
-Nef
-NCp7
b) mRNA Strategy
-gag splice
acceptor site
1

Fig. 7

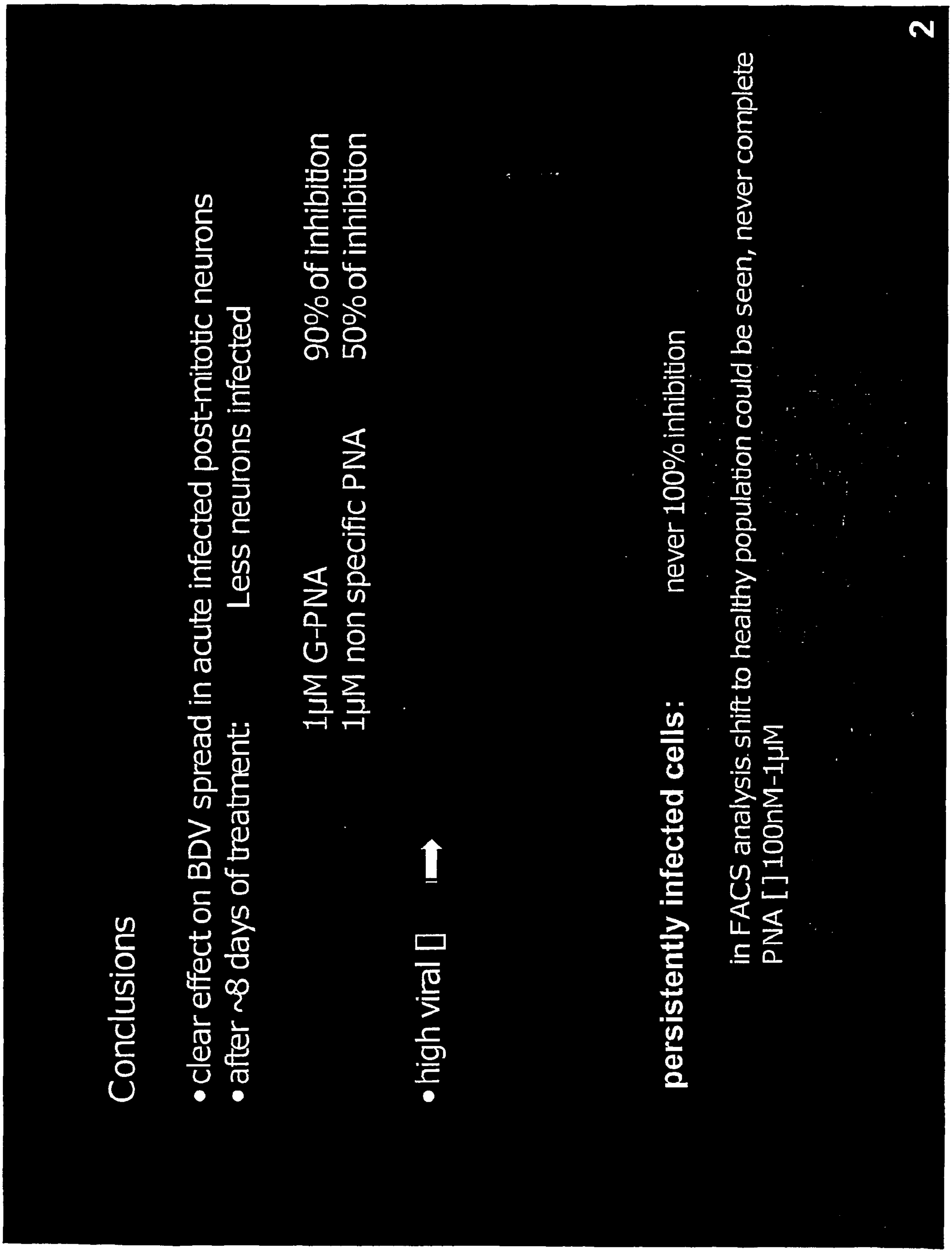
Conclusions
• clear effect on BDV spread in acute infected post-mitotic neurons
• after ~8 days of treatment: Less neurons infected
1µM G-PNA 90% of inhibition
1µM non specific PNA 50% of inhibition
• high viral
persistently infected cells: never 100% inhibition
in FACS analysis shift to healthy population could be seen, never complete
PNA [ ] 100nM-1µM
2

Fig. 8

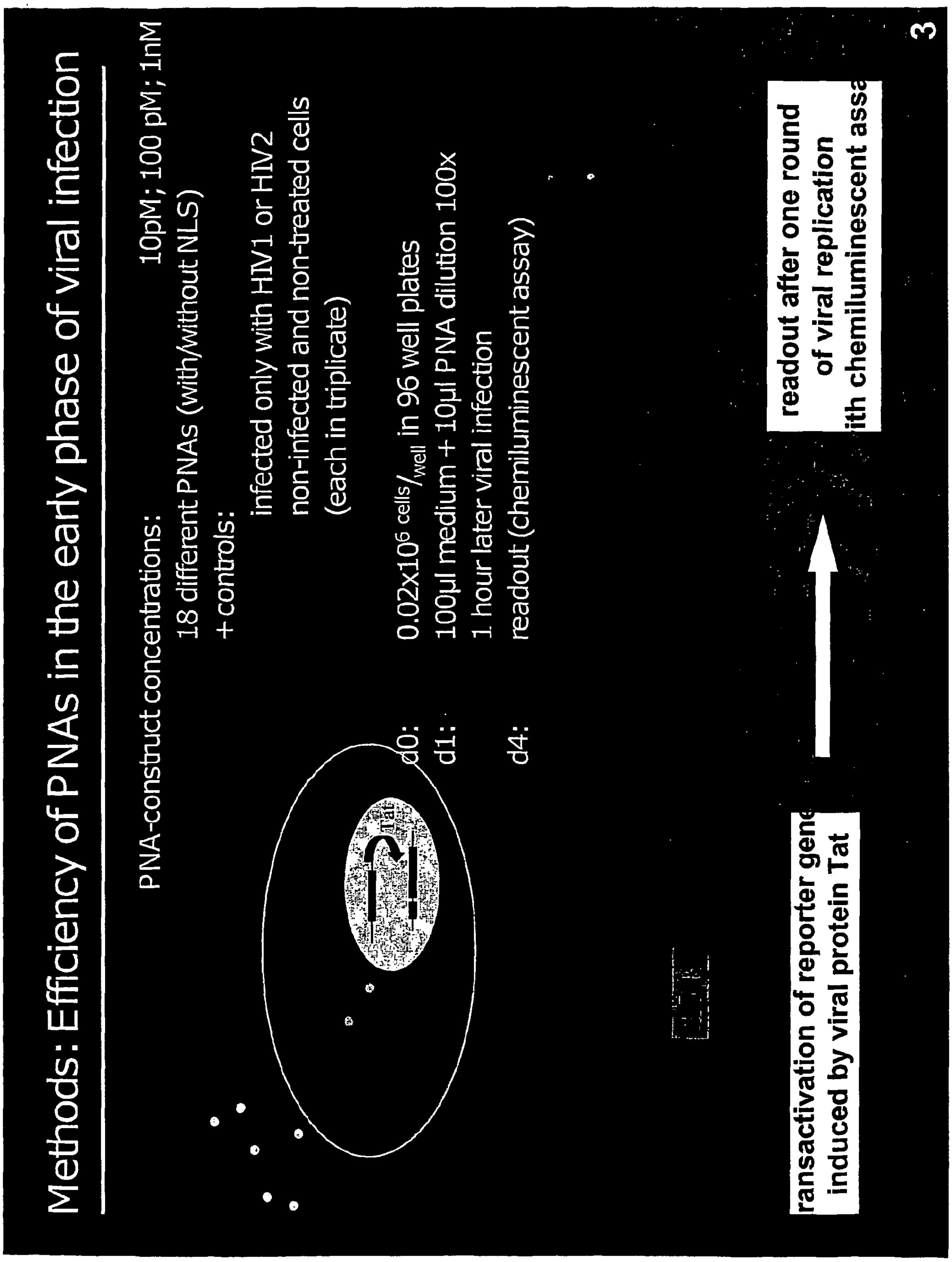
Methods: Efficiency of PNAs in the early phase of viral infection
PNA-construct concentrations: 10pM; 100 pM; 1nM
18 different PNAs (with/without NLS)
+ controls:
infected only with HIV1 or HIV2
non-infected and non-treated cells
(each in triplicate)
d0: 0.02x10^6 cells/well in 96 well plates
d1: 100µl medium + 10µl PNA dilution 100x
1 hour later viral infection
d4: readout (chemiluminescent assay)
Tat
ransactivation of reporter gen
induced by viral protein Tat
readout after one round
of viral replication
ith chemiluminescent ass
3

Fig. 9

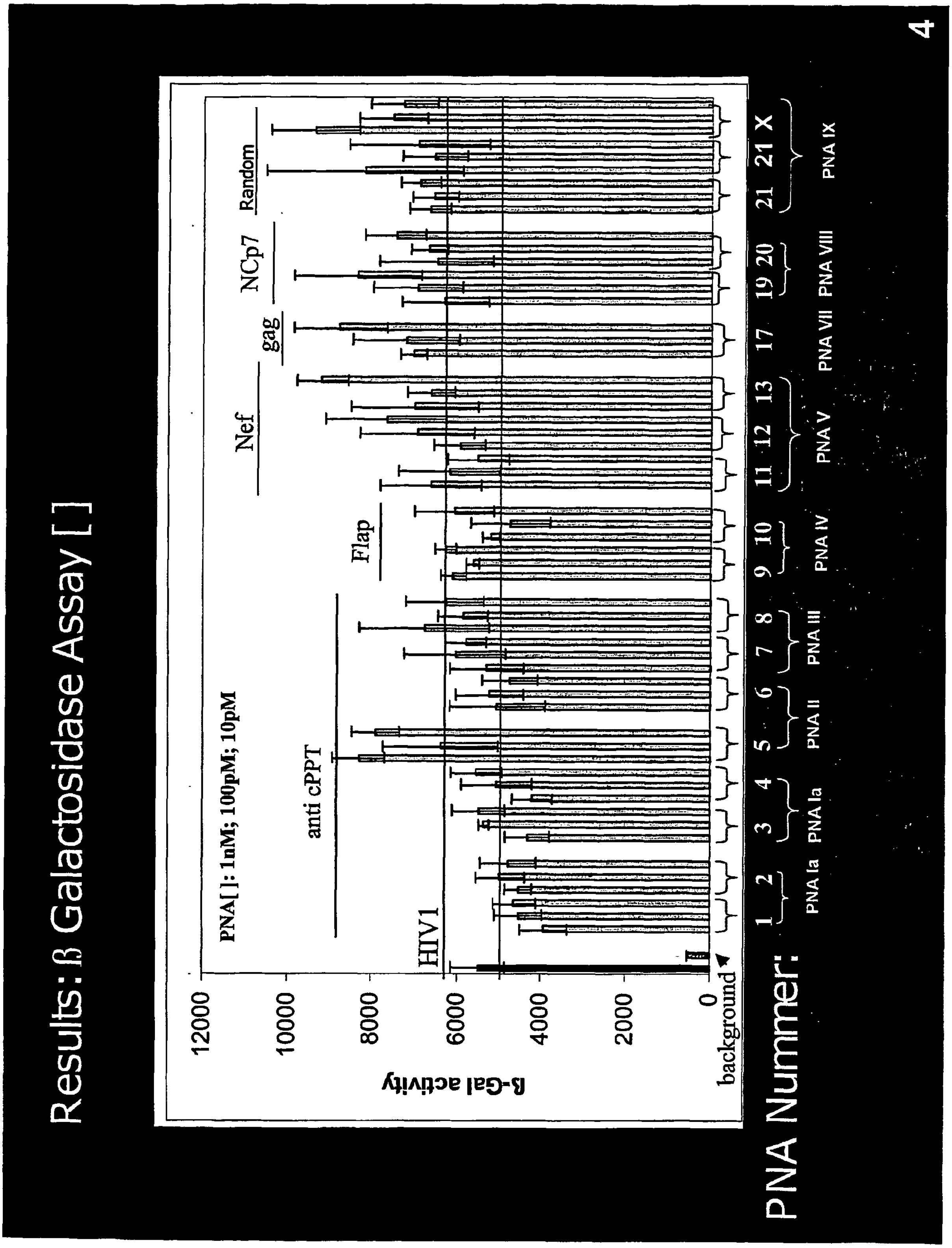
Results: ß Galactosidase Assay [ ]
PNA[ ]: 1nM; 100pM; 10pM
anti cPPT
Flap
Nef
gag
NCp7
Random
HIV1
ß-Gal activity
12000
10000
8000
6000
4000
2000
0
background
PNA Nummer:
1 2 3 4 5 6 7 8 9 10 11 12 13 17 19 20 21 21 X
PNA Ia
PNA Ia
PNA II
PNA III
PNA IV
PNA V
PNA VII
PNA VIII
PNA IX
4

Fig. 10

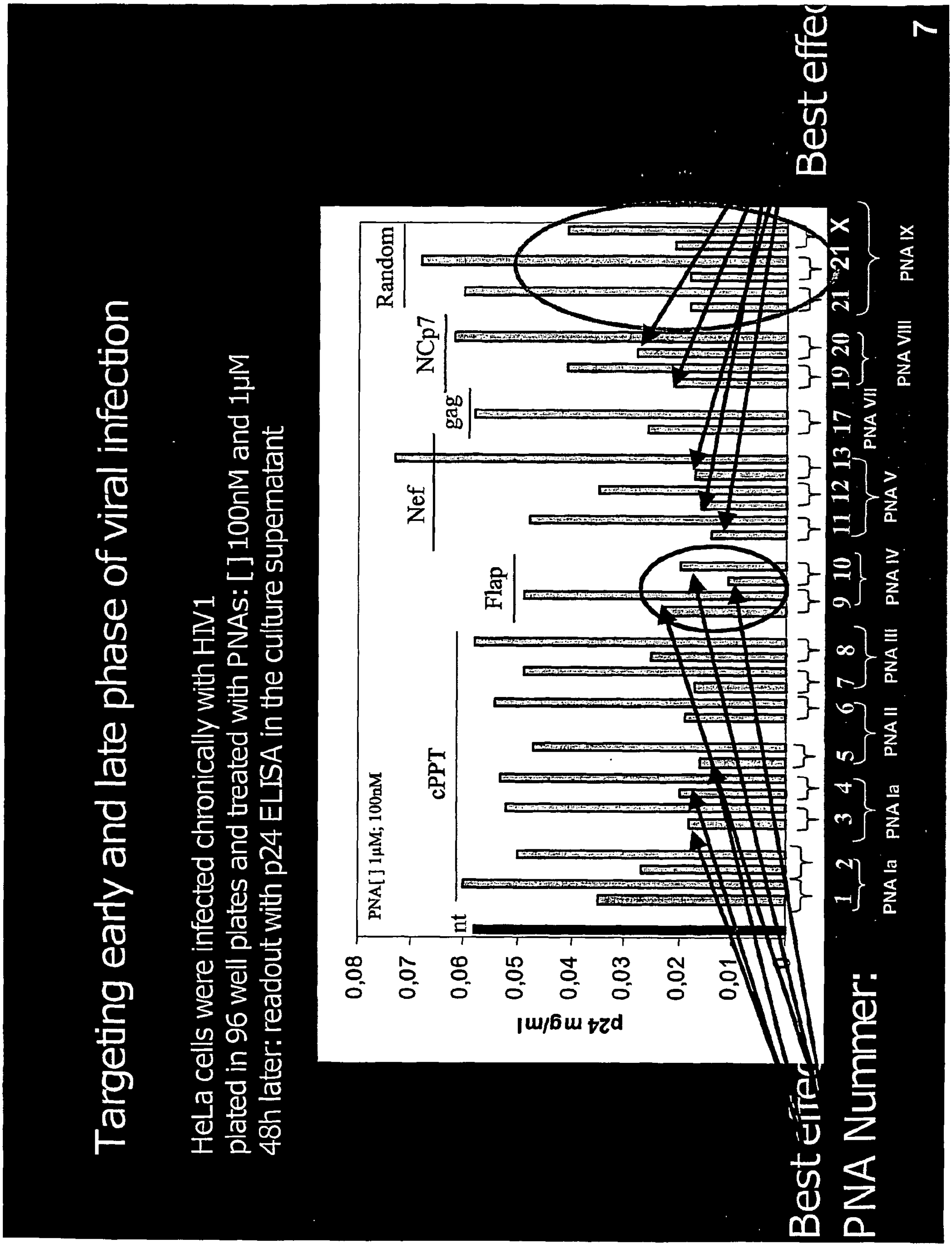
Targeting early and late phase of viral infection
HeLa cells were infected chronically with HIV1
plated in 96 well plates and treated with PNAs: [ ] 100nM and 1µM
48h later: readout with p24 ELISA in the culture supernatant
p24 mg/ml
0,08
0,07
0,06
0,05
0,04
0,03
0,02
0,01
PNA[ ] 1µM; 100nM
nt
cPPT
Flap
Nef
gag
NCp7
Random
PNA Nummer:
1 2 3 4 5 6 7 8 9 10 11 12 13 17 19 20 21 21 X
PNA Ia
PNA Ia
PNA II
PNA III
PNA IV
PNA V
PNA VII
PNA VIII
PNA IX
7

Fig. 11

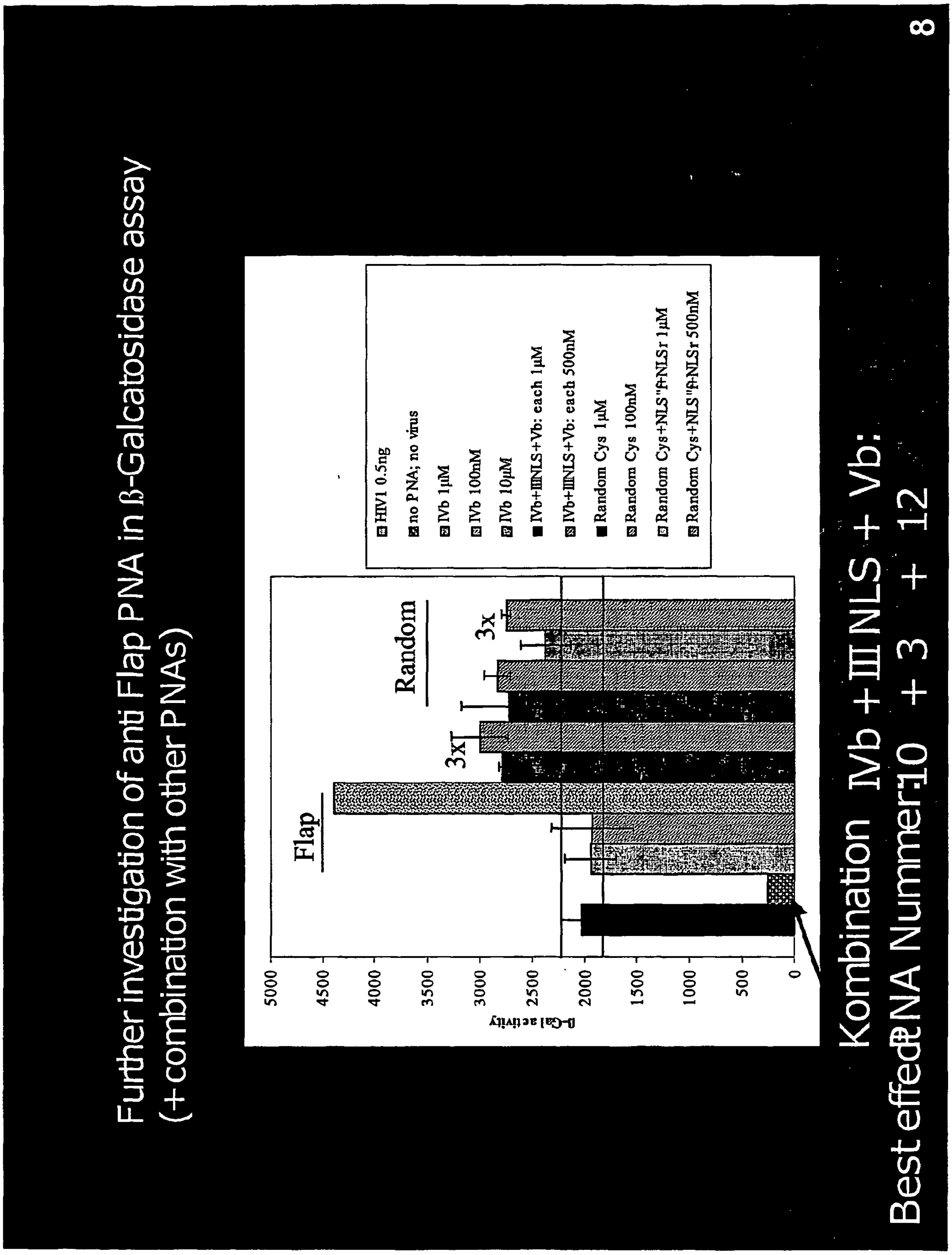

Further investigation of anti Flap PNA in ß-Galcatosidase assay
(+combination with other PNAs)
ß-Gal activity
5000
4500
4000
3500
3000
2500
2000
1500
1000
500
0
Flap
Random
3x
3x
HIV1 0.5ng
no PNA; no virus
IVb 1µM
IVb 100nM
IVb 10µM
IVb+IIINLS+Vb: each 1µM
IVb+IIINLS+Vb: each 500nM
Random Cys 1µM
Random Cys 100nM
Random Cys+NLS"f+NLSr 1µM
Random Cys+NLS"f+NLSr 500nM
Kombination IVb + III NLS + Vb:
Best effect PNA Nummer: 10 + 3 + 12
8


Fig. 12

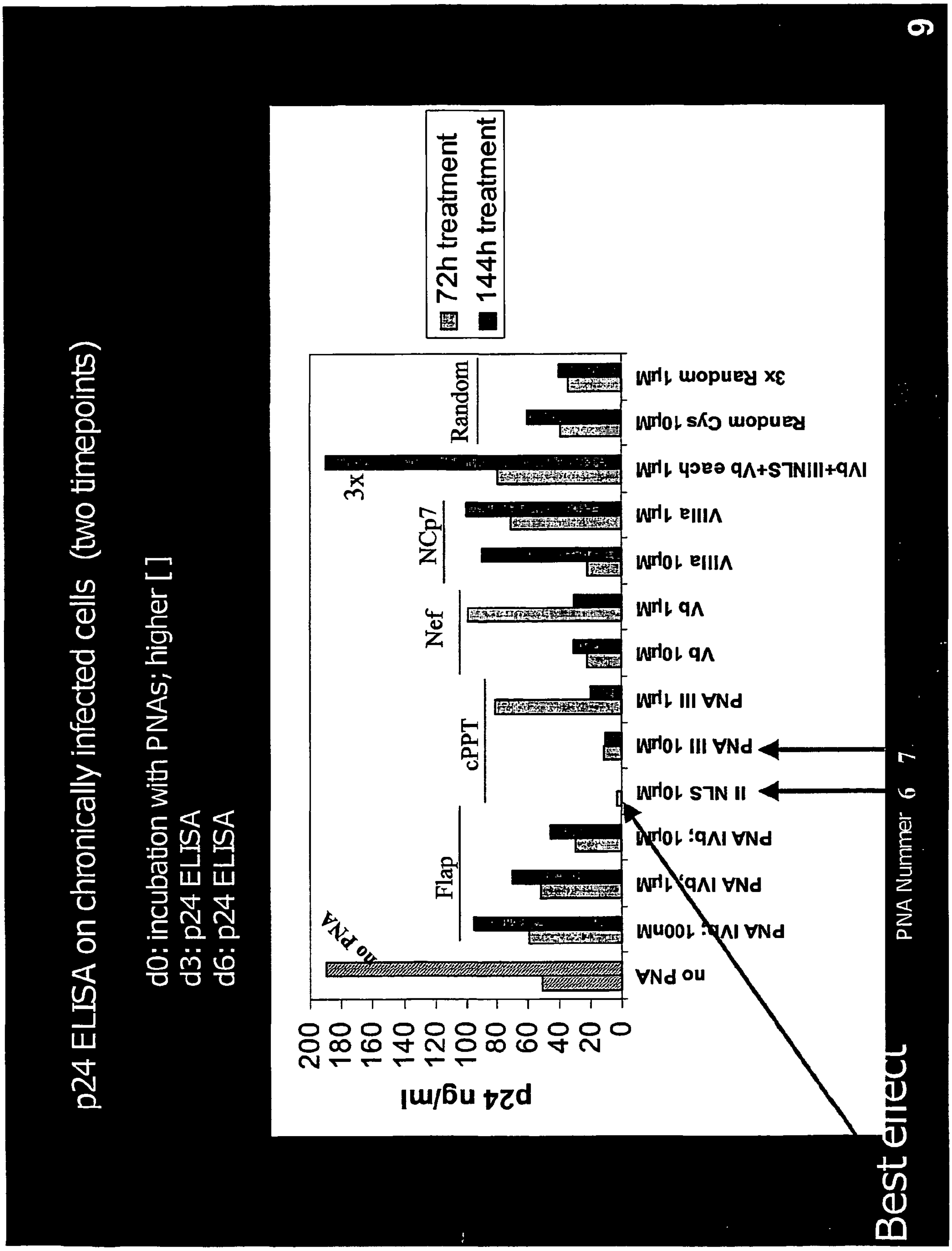
p24 ELISA on chronically infected cells (two timepoints)
d0: incubation with PNAs; higher [ ]
d3: p24 ELISA
d6: p24 ELISA
p24 ng/ml
200
180
160
140
120
100
80
60
40
20
0
72h treatment
144h treatment
no PNA
Flap
cPPT
Nef
NCp7
3x
Random
no PNA
PNA IVb; 100nM
PNA IVb; 1µM
PNA IVb; 10µM
II NLS 10µM
PNA III 10µM
PNA III 1µM
Vb 10µM
Vb 1µM
VIIIa 10µM
VIIIa 1µM
IVb+IIINLS+Vb each 1µM
Random Cys 10µM
3x Random 1µM
PNA Nummer 6 7
Best effect
9

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0105432 A
- DE 19933492


### In der Beschreibung aufgeführte Nicht-Patentliteratur


- **MCSHAN et al.** *JBC,* 1992, vol. 267, 5712-5721
- **LEE et al.** *Biochemistry,* 1998, vol. 37, 900-910
- **NIELSEN et al.** *Science,* 1991, vol. 254, 1497-1500
- **DEROSSI et al.** *Trends Cell Biol.,* 1988, vol. 8, 84-87
- **POOGA et al.** *The Faseb Journal,* 1998, vol. 12, 68 ff
- **MERRIFIELD.** *J. Am. Chem. Soc.,* 1963, vol. 85, 2149
- **RIETSCH ; BECKWITH.** *Annu. Rev. Genet.,* 1998, vol. 32, 163-84
- **RATNER et al.** *Nature,* 1985, vol. 313, 277-284
- **SHIRAMIZU et al.** *Cancer Res.,* 1994, vol. 54, 2069-2072
- **VON BRITTEN ; KOHNE.** *Science,* 1986, vol. 161 (3841), 529-540